# EUROPEAN PATENT SPECIFICATION

(11) **EP 1 849 409 B1**
(45) Date of publication and mention of the grant of the patent: **02.06.2010**
(21) Application number: 07106721.9
(22) Date of filing: 23.04.2007
(51) Int. Cl.: A61B 5/0215

(54) **Sensor and guidewire assembly**
Sensor und Führungsdrahtanordnung
Ensemble de capteur et fil-guide

(30) Priority: 28.04.2006 SE 0600943
(43) Date of publication of application: 31.10.2007
(73) Proprietor: RADI MEDICAL SYSTEMS AB, 754 50 Uppsala (SE)
(72) Inventor: Smith, Leif, 756 52 Uppsala (SE); von Malmborg, Pär, 755 97 Uppsala (SE); Düring, Erik, 753 30 Uppsala (SE)
(74) Representative: Holmberg, Nils Anders Patrik

(56) References cited:
- EP-A- 0 925 803
- WO-A-88/00810
- US-A- 5 226 423
- US-A1- 2001 051 769
- US-A1- 2003 023 190

## Description

### Field of the Invention

The invention relates generally to sensors mounted on guide wires for intravascular measurements of physiological variables in a living body, and in particular to the design of such sensor guide wires.

### Background of the Invention

Sensor and guide wire assemblies in which a sensor, adapted for measurements of physiological variables in a living body, such as blood pressure and temperature, is mounted at a distal portion of a guide wire are known.

For example, the U.S. Patent No. Re. 35,648, which is assigned to the present assignee, discloses a sensor and guide wire assembly comprising a sensor element, an electronic unit, signal transmitting cables connecting the sensor element to the electronic unit, a flexible tube having the signal cables and the sensor element disposed therein, a solid metal wire, and a coil attached to the distal end of the solid wire. The sensor element comprises a pressure sensitive device, e.g. a membrane, with piezoresistive elements electrically connected in a Wheatstone bridge-type of arrangement mounted thereon.

One physiological parameter that can be determined by use of a guide wire mounted pressure sensor is the so-called fractional flow reserve (FFR), which is used to assess the severity of a stenosis located somewhere in a coronary artery (see, e.g., "Coronary Pressure" by N. H. J. Pijls and B. De Bruyne, 2nd edition, Kluwer Academic Publishers, The Netherlands, 2000). The clinical value of FFR as a diagnostic tool is gaining increasing acceptance within the medical society, something which, in turn, has created a desire to apply the method in ever narrower arteries, i.e. further out in the coronary tree.

To measure a physiological parameter such as blood pressure at a measurement site located far out in a small and tortuous vessel put, however, very high requirements on the mechanical characteristics of the guide wire that carries the pressure sensor. In, for example, the U.S. Patent No. 5,226,423, which is assigned to the assignee of the present patent specification, a sensor guide wire is disclosed, in which a solid wire, which constitutes the core of the sensor guide, has been divided into a plurality of sections and each of the sections has a different thickness and thereby a different flexibility. A large flexibility of the sensor guide is advantageous in that it allows the sensor guide to be introduced into small and tortuous vessels. It should, however, also be recognized that if the core wire is too flexible, it would be impossible to push the sensor guide forward into the vessels, i.e. the sensor guide wire must possess a certain stiffness, torqueability, and "pushability".

To summarize: the desire to measure physiological variables such as blood pressure and temperature further out in the coronary tree has put the manufacturers of guide wire mounted sensors in a dilemma, because this urge implies that the torqueability and stiffness of the guide wire should be increased, which most easily can be accomplished if the diameter of a core wire arranged inside the sensor guide wire is increased. The core wire diameter is, however, limited by the outer diameter of the sensor guide wire, and this outer diameter cannot be increased without jeopardizing the compatibility with other interventional devices, such as different kinds of catheters which are threaded over the sensor guide wire in order to treat the stenosis that was diagnosed by the sensor and guide wire assembly. Ultimately, the diameters of all interventional devices are, however, apparently limited by the diameters of the narrow arteries of the peripheral coronary tree - if anything there is consequently a desire to reduce the outer diameter of a sensor guide wire. On the other hand, increasing the core wire diameter without a corresponding increase of the outer diameter of the sensor guide would leave less space available for the signal transmitting cables that extend in the interior of the sensor guide.

The electrical signal cables, which provide the sensor with the electrical excitation energy necessary to operate the Wheatstone bridge and which transfer the output signals from the sensor to an external display unit, are thin and sensitive members, each of which requires its own electrical insulation. An alternative arrangement for transmitting the sensor signals is described in the U.S. Patent No. 6,106,486, which is assigned to the present assignee and wherein it is suggested to transmit the sensor signals in conductors which in the form of layers of electrically conductive material extend concentrically over the circumference of the guide wire, and wherein the outermost conductive layer is covered with an insulating layer. In the published U.S. Patent application 2003/0028128 A1, a sensor guide is described wherein a signal conductor is disposed concentrically in the central lumen of a thick-walled tube; and the published U.S. Patent application 2003/0220588 A1 discloses a similar arrangement, comprising at least two signal conductors arranged within the central lumen of a thick-walled tube. These two applications, which are assigned to the present assignee, state that the advantage of substituting a thick-walled tube for a core wire is, inter alia, that the conductors, when arranged in the lumen of the thick-walled tube, are better protected against damages caused by the handling of the sensor guide. Damage on an electrical signal cable or, perhaps more likely, on the electrical insulation surrounding the cable can lead to unreliable performance or even short-circuit of the sensor. It can further be noted that the sensor guides disclosed in the two applications are provided with outer insulating layers which can be made from different kinds of polymers. Sensor guide wires comprising a thick-walled tube having a lumen in which a number of signal conductors are arranged, or sensor guide wires comprising a number of concentric conductive layers are, however, considered to represent very special designs of sensor guide wires; and those types fall outside the scope of the present invention as defined by the claims.

The latter two patent applications as well as several other known sensor guide wires, e.g. the sensor guide wire disclosed in the U.S. Patent No. 5,715,827 to Corl et al., exhibits a design that includes a distal coil spring extending from the distal tip of the sensor guide to a sensor housing, inside which the sensor element is arranged, and a proximal coil spring extending between the housing and a proximal tube. The proximal coil spring is provided for improving the manoeuvrability of the sensor guide wire, but may also put further limitations on the maximum dimensions of a core wire disposed therein.

Document WO 88/00810 discloses a sensor guidewire assembly according to the preamble of claim 1.

A general object of the present invention is to provide an improved design for a sensor and guide wire assembly, which enhances the manoeuvrability of the sensor guide wire and, at the same time, reduces the risk of electrical failure of the signal transmitting cable(s) arranged in the sensor guide wire.

### Summary of the Invention

The above-mentioned object is achieved by the present invention according to the independent claims.

Preferred embodiments are set forth in the dependent claims.

Embodiments of the present invention are directed to a sensor and guide wire assembly comprising a sensor element arranged in a sensor guide wire having a distal tip and comprising a core wire, a proximal tube, and at least one electrical signal transmitting cable. The sensor element is mounted at a distal portion of the core wire, and is connected to the one or more electrical signal cables, which extend from the sensor element to the proximal end portion of the sensor guide wire, where each electrical cable is connected to a conductive member. The conductive members are electrically insulated from each other by insulating members, and are arranged longitudinally spaced from each other at the proximal end portion of the sensor guide wire, so as to form a male connector for further connection to a corresponding female connector of an external signal conditioning and display unit. Although not necessary prerequisites for the present invention, the sensor guide wire can further be fitted with a jacket as well as a distal coil, which surrounds the distal portion of the core wire and extends between the distal tip and the jacket. The sensor element is disposed inside the jacket, and is through a window in the jacket in fluid communication with the surrounding medium, e.g. blood.

According to embodiments of the present invention, a sensor guide wire comprises a polymer layer, which is provided in the vicinity of a sensor element and encloses a portion of a core wire and a number of signal transmitting cables. If the sensor guide wire is equipped with a sensor housing in the form of a jacket or sleeve, the polymer layer extends preferably between the jacket and a proximal tube. If no jacket is present, the polymer layer can extend from the proximal tube to a distal coil, or, if no distal coil is provided, all the way to the distal tip of the sensor guide wire.

From one aspect, a polymer layer can be regarded as a replacement for a proximal coil spring, and has the advantage that it easily can be made thinner than a conventional coil spring, thereby providing the possibility to increase the outer diameter of the core wire portion enclosed by this polymer layer. As previously discussed, a larger core wire diameter implies a higher torqueability and thereby improved manoeuvrability of the sensor guide wire. On the other hand, if the core wire diameter is left unchanged, more space can be provided for the signal transmitting cables, which, in turn, may involve the possibility of taking different kinds of measures to protect the electrical signal cables. The thickness of the electrical insulation surrounding the cables can, for example, be increased.

Further, in contrast to a coil spring, which inherently is permeable to bodily fluids such as blood, a polymer layer can easily be made essentially impermeable to bodily fluids. An impermeable outer layer entails the advantage that the insulating requirements on the thin signal transmitting cables can be reduced as no electrically conductive fluid will be present between the cables.

A soft polymer layer will also be very unlikely to damage the thin and sensitive signal cables, because, for example, there is no risk that the cables are squeezed between a core wire and an inelastic outer member such as a proximal coil spring.

A polymer layer can be provided as a tube or sleeve that encloses a portion of a core wire and a number of signal transmitting cables extending along this portion of the core wire, or a polymer layer can be coated onto a core wire, with the signal cables being embedded in the polymer layer.

In preferred embodiments of the present invention, a polymer layer can comprise a low-friction material and/or a hydrophilic agent for reducing the friction between the outer surface of the sensor guide wire and a vessel wall as the sensor guide wire is advanced through sharp bends in narrow and tortuous vessels. The low-friction material and/or the hydrophilic agent can be applied as a coating on the surface of the polymer layer, or can be incorporated in the polymer layer itself. Another possibility is that the polymer layer comprises or consists of a low-friction and/or hydrophilic polymeric material.

### Brief Description of the Drawings

Fig 1 is a schematic illustration of a sensor and guide wire assembly according to the prior art.
Fig. 2 is a schematic illustration of a first embodiment of a sensor and guide wire assembly according to the present invention, wherein a sensor guide wire comprises a polymer layer extending between a proximal tube and a jacket.
Fig. 3 is a schematic illustration of a second embodiment of a sensor and guide wire assembly according to the present invention, wherein a sensor guide wire comprises a polymer layer extending between a proximal tube and a distal coil spring.
Fig. 4 shows a cross-section of a third embodiment of a sensor and guide wire assembly according to the present invention, wherein a sensor guide wire comprises a number of signal transmitting cables which are embedded in a polymer layer.

### Detailed Description of Preferred Embodiments

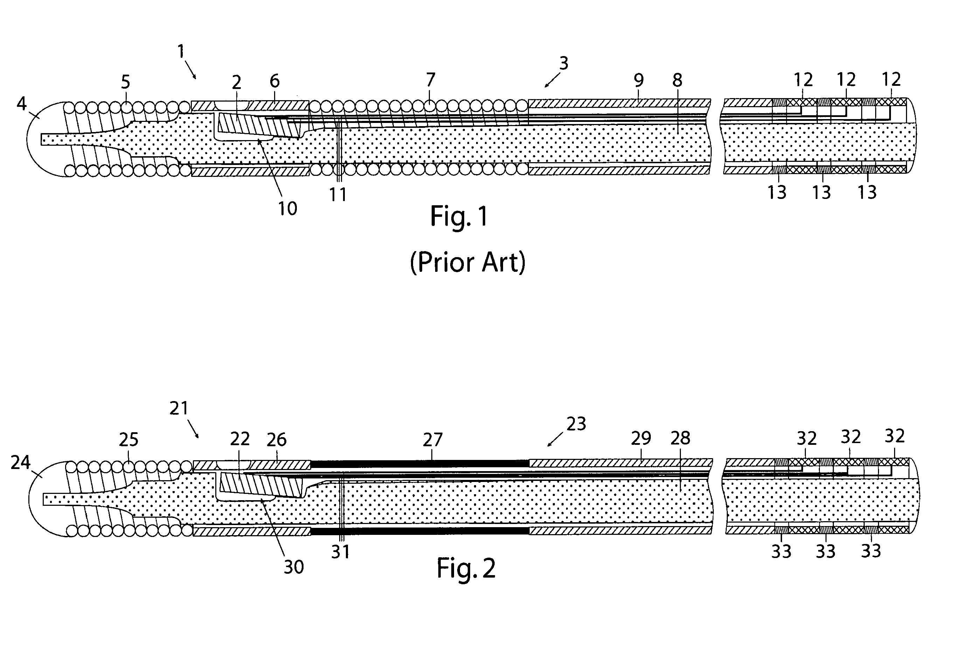

Fig. 1 illustrates schematically the design of a sensor and guide wire assembly 1 according to the prior art. The sensor and guide wire assembly 1 comprises a sensor element 2, which is arranged in a distal portion of a sensor guide wire 3. More specifically, the sensor guide wire 3 comprises a distal tip 4, a distal coil spring 5, a jacket or sleeve 6, a proximal coil spring 7, a core wire 8, and a proximal tube 9. The distal coil spring 5 is attached to the distal tip 4 and extends to the jacket 6, which serves as a housing for the sensor element 2. The proximal coil spring 7 extends between the jacket 6 and the proximal tube 9. The distal coil spring 5, the jacket 6, the proximal coil spring 7 and the proximal tube 9 are all tubular members having essentially equal outer diameters and surrounding different consecutive portions of the core wire 8. The sensor element 2 is mounted in a recess 10 in a distal portion of the core wire 8, and is through a window in the jacket 6 in communication with the medium, e.g. blood, surrounding the sensor and guide wire assembly 1. The sensor and guide wire assembly 1 comprises further a number of signal transmitting cables 11, the distal ends of which are electrically connected to the sensor element 2 and which extend along the core wire 8 to the proximal end portion of the sensor and guide wire assembly 1, where each signal transmitting cable 11 is electrically connected to a conductive member 12. The conductive members 12 are electrically insulated from each other by insulating members 13, so as to form a male connector adapted for connection to a corresponding female connector of an external signal conditioning and display unit (not shown in Fig. 1).

From Fig. 1 it may be appreciated that the wall thickness of the proximal coil spring 7 is larger than the wall thickness of the proximal tube 9. (The wall thickness of the coil spring 7 is given by the diameter of the metal thread that has been wound to form the coil spring 7.) The present assignee manufactures and sells a guide wire mounted pressure sensor under the registered trademark PressureWire®, which incorporates the essential features of the sensor and guide wire assembly shown in Fig. 1, and practical experience has revealed that a comparatively larger wall thickness of a proximal coil is essential to provide a sensor guide having the robustness (e.g. kink resistance) necessary for different medical procedures, e.g. balloon catheterizations in which a catheter equipped with an inflatable balloon is advanced over the sensor guide wire. The space available for the core wire 8 and signal cables 11 is limited by the inner diameter of the proximal coil spring 7.

When a guide wire like sensor guide wire 3 is manoeuvred through the tortuous arteries of a patient's coronary system, a coil spring, like proximal coil spring 7, will be bent, which means that small gaps will appear between the consecutive windings of the coil spring at the outer bending radius of the guide wire. Consequently, the sensor guide wire 3 is inherently permeable to the medium, e.g. blood, surrounding the sensor guide wire 3. In other words, bodily fluids such as blood will penetrate into the interior of the sensor guide 3, and will in particular be in contact with the signal cables 11. Each of the signal cables 11 is therefore individually insulated by a thin tubing or coating of an electrically non-conductive material that encloses the signal cable along its length. Any damage on this insulating layer will lead to unreliable performance of the sensor and guide wire assembly 1, and can also cause a short-circuit of the sensor element 2. Needless to say, the requirements on these insulating layers are consequently severe.

In Fig. 2 a first embodiment of a sensor and guide wire assembly 21 according to the present invention is schematically illustrated. The sensor and guide wire assembly 21 comprises a sensor element 22, which is arranged in a distal portion of a sensor guide wire 23. More specifically, the sensor guide wire 23 comprises a distal tip 24, a distal coil spring 25, a jacket or sleeve 26, a core wire 28, and a proximal tube 29. The distal coil spring 25 is attached to the distal tip 24 and extends to the jacket 26, which serves as a housing for the sensor element 22. Unlike in a conventional design of a sensor guide wire, an example of which is shown in Fig. 1, the sensor guide wire 23 comprises further a polymer layer 27, which extends between the jacket 26 and the proximal tube 29. The distal coil spring 25, the jacket 26, the polymer layer 27 and the proximal tube 29 are all tubular members having essentially equal outer diameters and surrounding different consecutive portions of the core wire 28. The sensor element 22 is mounted in a recess 30 in a distal portion of the core wire 28, and is through a window in the jacket 26 in communication with the medium, e.g. blood, surrounding the sensor and guide wire assembly 21. The sensor and guide wire assembly 21 comprises further a number of signal transmitting cables 31, the distal ends of which are electrically connected to the sensor element 22 and which extend along the core wire 28 to the proximal end portion of the sensor and guide wire assembly 21, where each signal transmitting cable 31 is electrically connected to a conductive member 32. The conductive members 32 are electrically insulated from each other by insulating members 33, so as to form a male connector adapted for connection to a corresponding female connector of an external signal conditioning and display unit (not shown in Fig. 2).

A comparison between the sensor and guide wire assembly 1 of Fig. 1 and the sensor and guide wire assembly 21 of Fig. 2 reveals that the polymer layer 27 of the sensor assembly 21 can be regarded as a replacement for the proximal coil spring 7 of the sensor assembly 1. In a sensor and guide wire application, there are, however, at least two important differences to be noted between a polymer layer and a coil spring:

First, a thin-walled coil spring having, for example, an outer diameter of about 0.36 mm (0.014 inches) and an inner diameter of about 0.25 mm (0.010 inches) has a bending rigidity which is negligible in comparison with the bending rigidity of a polymer layer in the form of a nylon or polyimide tube with approximately the same dimensions. As previously discussed, a rather high bending rigidity and thereby kink resistance of an outer member, such as a polymer tube, of a sensor guide wire is necessary, or at least advantageous, in certain intravascular medical procedures such as balloon catheterizations, and contributes also significantly to the overall stiffness, torqueability and pushability of the sensor guide wire. Consequently, as also is to be seen from a comparison between Fig. 1 and Fig. 2, the polymer layer 27 of the sensor guide wire 23 of Fig. 2 can be made thinner than the proximal coil spring 7 of the sensor guide wire 3 of Fig. 1 without deteriorating the overall medical performance of the sensor guide wire 23. This advantageous achievement is accompanied by the possibility to increase the diameter of a core wire enclosed by such a polymer layer; and the diameter of the core wire 28 in the sensor guide wire 23 of Fig. 2 has accordingly been made larger than the diameter of the core wire 8 in the sensor guide wire 3 of Fig. 1.

Second, a coil spring like proximal coil spring 7 of the sensor guide wire 3 of Fig. 1 is essentially permeable to bodily fluids such as blood. During use of the sensor and guide wire assembly 1 such fluids will therefore penetrate into the interior of the sensor guide wire 3, and will in particular be present around the signal transmitting cables 11. Each of the signal cables 11 is therefore provided with a separate insulating layer, but there is nevertheless a constant risk that such an insulating layer is damaged, for example if a signal cable 11 is squeezed between the core wire 8 and the proximal coil spring 7. Damage on the insulating layer will affect the output from the sensor element 2, and will thereby lead to unreliable performance of the sensor and guide wire assembly 1. In contrast, the polymer layer 27 of the sensor guide wire 23 of Fig. 2 is essentially impermeable to bodily fluids such as blood. This impermeable feature of the polymer layer 27 in combination with the fact that the proximal portion of the sensor element 22 is embedded in an impermeable material, such as glue, epoxy or silicone (not shown in Fig. 2), ensures that during use of the sensor and guide wire 21 bodily fluids will not penetrate into the interior of the sensor guide wire 23, and will in particular not be present around the signal transmitting cables 31. The insulating requirements on the individual insulating layers enclosing the signal transmitting cables 31 are therefore less severe, which may lower the costs for manufacturing a sensor and guide wire assembly and contributes positively to the reliability and durability of the sensor and guide wire assembly.

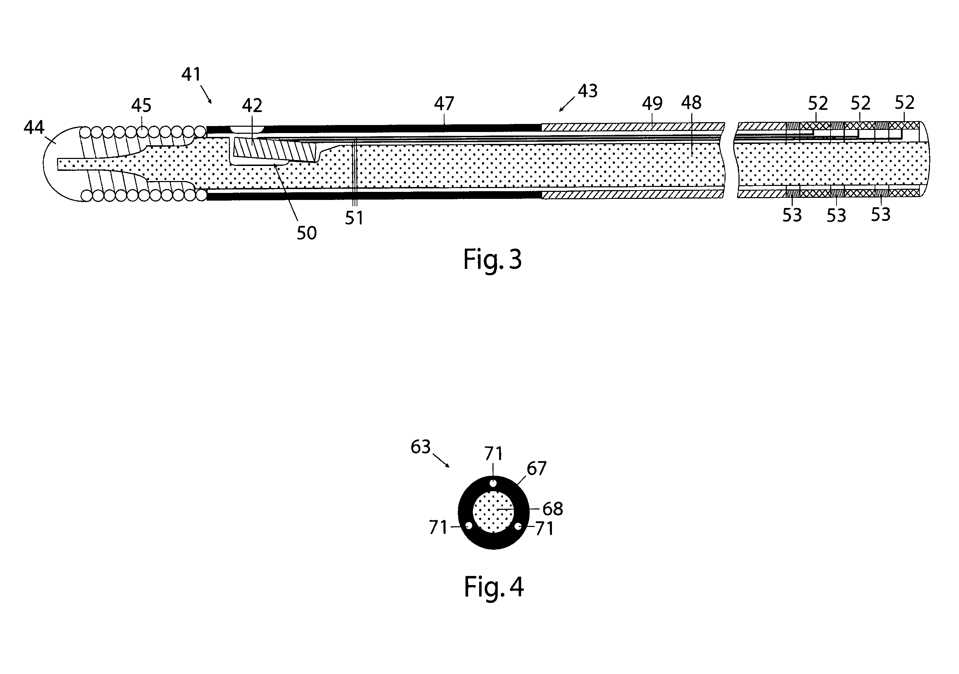

In Fig. 3 a second embodiment of a sensor and guide wire assembly 41 according to the present invention is schematically illustrated. The sensor and guide wire assembly 41 comprises a sensor element 42, which is arranged in a distal portion of a sensor guide wire 43. More specifically, the sensor guide wire 43 comprises a distal tip 44, a distal coil spring 45, a polymer layer 47, a core wire 48, and a proximal tube 49. The distal coil spring 45 is attached to the distal tip 44 and extends to the polymer layer 47. In this embodiment, the sensor element 42 is enclosed by the polymer layer 47, which also serves as a housing for the sensor element 42. The distal coil spring 45, the polymer layer 47 and the proximal tube 49 are all tubular members having essentially equal outer diameters and surrounding different consecutive portions of the core wire 48. The sensor element 42 is mounted in a recess 50 in a distal portion of the core wire 48, and is through a window in the polymer layer 47 in communication with the medium, e.g. blood, surrounding the sensor and guide wire assembly 41. The sensor and guide wire assembly 41 comprises further a number of signal transmitting cables 51, the distal ends of which are electrically connected to the sensor element 42 and which extend along the core wire 48 to the proximal end portion of the sensor and guide wire assembly 41, where each signal transmitting cable 51 is electrically connected to a conductive member 52. The conductive members 52 are electrically insulated from each other by insulating members 53, so as to form a male connector adapted for connection to a corresponding female connector of an external signal conditioning and display unit (not shown in Fig. 3).

Thus, the essential difference between the second embodiment of Fig. 2 and the third embodiment of Fig. 3 is that a separate housing in the form of a jacket or sleeve for the sensor element has been dispensed with in the third embodiment. Since a jacket, which usually is made from a metal, is an essentially stiff element compared to a polymer layer or a coil spring, the sensor guide wire 43 of Fig. 3 will have more regular bending characteristics over its distal portion than the sensor guide wire 23 of Fig. 2. The sensor assembly 41 of Fig. 3 comprises also fewer parts than the sensor assembly 21 of Fig. 2, and is consequently easier and cheaper to assemble. It also within the scope of the present invention to provide a sensor and guide wire assembly in which a polymer layer extends between a distal tip of a sensor guide wire and a proximal tube, i.e. it is possible to omit a distal coil spring. Such a sensor guide would exhibit very regular bending characteristics over its distal portion, and would also be easy and cheap to manufacture. When a polymer layer is applied as a sleeve or tube, a small gap can be maintained between the sleeve or tube and the signal cables enclosed by this sleeve or tube. The signal cables are then not tightly squeezed between the sleeve or tube and the core wire, but have a certain freedom to move, which may enhance the durability of the sensor and guide wire assembly. A fourth embodiment of a sensor and guide wire assembly according to the present invention comprises a sensor guide wire 63 having a cross-section which is schematically illustrated in Fig. 4. The sensor guide wire 63 comprises a core wire 68, three signal transmitting cables 71 and a polymer layer 67. Rather than being arranged around the signal transmitting cables 71 - as in the previous embodiments -, the polymer layer 67 has been applied as a coating 67 on a section of the core wire 68, with the three signal cables 71 being embedded in the polymer coating 67. With this arrangement, the signal cables 71 can be produced without a separate insulating layer on each signal cable 71, because the polymer coating will electrically insulate each of the signal transmitting cables 71. When the signal cables are embedded in a polymer layer, the diameter of the core wire can be increased in comparison with an arrangement where a polymer layer in the form of a tubing surrounds the signal cables. The stiffness, torqueability and pushability of a sensor guide wire is to a very large extent depending on the characteristics of a core wire disposed in the sensor guide wire, and a larger diameter of the core wire will thereby improve the overall mechanical properties of the sensor guide wire. The latter statement, which is valid for all the embodiments presented in the present specification, includes also the so-called traceability, which relates to the capability of a sensor guide wire to serve as a guide for other interventional devices, such as catheters, which are treaded onto and advanced over the sensor guide wire, without any kinks appearing on the sensor guide wire. Another important property of a sensor guide wire is a low tendency to flip, i.e. that the distal end does not respond immediately to a turn of the proximal end of the sensor guide wire but flips in an uncontrolled way after a number of turns of the proximal end. Also this property can be improved with a polymer layer, and here the joint between a proximal tube and the polymer layer seems to be the crucial parameter. Generally, a sensor guide wire comprising a polymer layer and a proximal tube exhibits a lower tendency to flip than a sensor guide wire comprising a proximal tube and a coil spring.

The present invention relates to sensor and guide wire assemblies, which typically have a length ranging from 1 m to 3 m. The most common commercially available sensor guide wires have an outer diameter of about 0.36 mm (0.014 inches), and core wire diameters between 0.1 mm to 0. 25 mm, typically with tapered distal portions.

The core wires can be made from stainless steel or a super-elastic alloy, e.g. a NiTi-alloy, or a shape-memory metal such as Nitinol. A polymer layer, which can be applied as a tubing, can have a wall thickness between about 0.025 mm and about 0.075 mm, and one or several polymers can be combined into one tubing. Examples of suitable polymers are polyimide and nylon. A signal transmitting cable or lead, having one or several strands, can have a diameter between about 0.02 mm and 0.04 mm, with a polymer insulation having a thickness of about 0.002 mm to about 0.012 mm. As used herein, a signal (transmitting) cable or lead is considered to be a thin electrically conductive thread, which can be arranged along the length of a core wire. To exemplify, a conductor provided in the form of a concentric layer of electrically conductive material is not considered to fall within the present definition of a signal (transmitting) cable or lead; and a sensor and guide wire assembly comprising such a conductor falls consequently outside the scope of the present invention. Further, as used herein, a core wire is considered to be a solid wire which generally is arranged in the centre of a sensor guide wire and whose mechanical properties, e.g. torqueability and stiffness, determine the general mechanical properties of the sensor guide wire. To exemplify, a hollow core having a lumen in which electrical leads can be arranged is not considered to fall within the present definition of a core wire; and a sensor and guide wire assembly comprising such a core wire falls consequently outside the scope of the present invention.

As was discussed above, the bending resistance of a polymer tube, which can serve as a polymer layer in a sensor guide wire, is much higher than the bending resistance of a corresponding coil spring, which typically can be arranged as a proximal coil spring in the manner shown in Fig. 1. Tests have, for example, shown that the bending resistance of a polyimide tube having an outer diameter of 0.325 mm and an inner diameter of 0.2165 mm is about 1.075 N/mm; and the bending resistance of nylon tube having the same dimensions is about 0.635 N/mm. These values can be compared with a bending resistance of only about 0.0115 N/mm for a coil spring made from stainless steel and having an outer diameter of about 0.35 mm and an inner diameter of about 0.25 mm. Thus, the bending resistance of a coil spring suitable for the present application is only about 1.1 percent and 1.8 percent of the bending resistances of a polyimide tube and a nylon tube, respectively, with approximately the same dimensions. A polymer tube will consequently contribute to a much larger extent to the, torqueability, stiffness and pushability of a sensor guide wire than a coil spring. Here, it should, however, be mentioned that a coil spring possesses certain other properties that may contribute in an advantageous way to the overall manoeuvrability of a sensor guide wire. A coil spring arranged at a distal portion of a guide wire can, for example, impart a quasi-additional degree of freedom to the distal portion of a core wire when the distal end of the guide wire encounters a sharp bend. In such case, the coil spring might stop moving when it enters the sharp bend, but the distal portion of the core wire will still be permitted to move within the coil spring, thus imparting additional torque to the end of the guide wire, forcing the distal end through the sharp bend. Consequently, from the discussion above it should be appreciated that a polymer layer and a coil spring are not interchangeable elements, because their mechanical characteristics, in particular as parts of a sensor guide wire, are completely different.

In accordance with an alternative embodiment of the present invention the polymer layer arranged proximally the sensor element instead is a metal tube, preferably made from stainless steel. The sensor housing may be made integral to the metal tube. The polymer layer arranged distally the sensor element, i.e. between the sensor element and the distal tip, is preferably made from a polymer.
As a result of mechanical performance analyses this alternative embodiment has proven good mechanical performance, e.g. with regard to torque transmittal between the proximal and the distal region, less angular lag during rotation has been identified, and more force is required to distally buckle the pressure wire.

In all of the embodiments described and discussed in conjunction with Figs. 2-4 above, the polymer layer can be combined with a low-friction material and/or a hydrophilic agent or material. A low-friction material or a hydrophilic agent or material can be applied as a coating on top of the polymer layer, or the low-friction material and/or the hydrophilic agent or material can be incorporated into the polymer layer. Another possibility is that the polymer layer comprises a mixture of a polymer and a hydrophilic agent or material and/or a low-friction material, or that the polymer layer consists of a hydrophilic polymer material and/or a low-friction material. Typical examples of low-friction materials would be materials made from polytetrafluoroethylene, e.g. Teflon®, while a hydrophilic coating can be based on a flouro-polymer. One suitable hydrophilic coating is hyaluronan, which is available from Biocoat, Inc. Other suitable hydrophilic coatings are available from Hydromer, Inc. The polymer layer can be reinforced with different structures of suitable materials other than polymers. As an example, the polymer layer can be braided with thin metal threads embedded in the polymer layer. Such thin metal threads can, for example, be arranged in a mesh structure. The core wire, the signal cables, and the polymer layer can advantageously be manufactured in one single extrusion process, i.e. the core wire, the signal cables, and the polymer layer are all co-extruded.

Although the present invention has been described with reference to specific embodiments, also shown in the appended drawings, it will be apparent for those skilled in the art that many variations and modifications can be done within the scope of the invention as described in the specification and defined with reference to the claims below.

## Claims

1. Sensor and guide wire assembly for intravascular measurements of a physiological variable in a living body, comprising a sensor element (22, 42) and a sensor guide wire comprising a core wire (28, 48) and at least one signal transmitting cable (31, 51) connected to the sensor element, said at least one signal transmitting cable (11,21,31) extends along the core wire to the proximal end portion, the sensor guide wire further comprises a proximal tube (29, 49), a distal tip (24, 44), and a polymer layer (27, 47) which encloses a portion of the core wire and said at least one signal transmitting cable, wherein the polymer layer extends between the distal tip and the proximal tube and **characterized in that** the signal transmitting cable is electrically connected to a conductive member (32, 52), so as to form a proximal male connector.

2. Sensor and guide wire assembly according to claim 1, **characterized in that** the polymer layer is provided as a tube or sleeve.

3. Sensor and guide wire assembly according to claim 1, **characterized in that** the polymer layer is provided as a coating.

4. Sensor and guide wire assembly according to claim 1, **characterized in that** the sensor guide wire further comprises a sensor housing (26) and the polymer layer extends between the sensor housing and the proximal tube.

5. Sensor and guide wire assembly according to claim 4, **characterized in that** the sensor housing is provided in the form of a jacket or sleeve.

6. Sensor and guide wire assembly according to claim 4, **characterized in that** the polymer layer constitutes the sensor housing.

7. Sensor and guide wire assembly according to claim 1, **characterized in that** the polymer layer proximally the sensor element instead is a metal tube.

8. Sensor and guide wire assembly according to claim 7, **characterized in that** metal tube is made from stainless steel.

9. Sensor and guide wire assembly according to claim 1, **characterized in that** the sensor guide wire further comprises a distal coil spring (25, 45) and the polymer layer extends between the distal coil spring and the proximal tube.

10. Sensor and guide wire assembly according to claim 1, **characterized in that** said at least one signal transmitting cable (71) is embedded in the polymer layer.

11. Sensor and guide wire assembly according to claim 1, **characterized in that** the polymer layer comprises a low-friction material.

12. Sensor and guide wire assembly according to claim 11, **characterized in that** the low-friction material is applied as a coating on the polymer layer.

13. Sensor and guide wire assembly according to claim 11, **characterized in that** the low-friction material is incorporated in the polymer layer.

14. Sensor and guide wire assembly according to claim 11, **characterized in that** the polymer layer consists of a low-friction material.

15. Sensor and guide wire assembly according to claim 1, **characterized in that** the polymer layer comprises a hydrophilic agent or material.

16. Sensor and guide wire assembly according to claim 15, **characterized in that** the hydrophilic agent or material is applied as a coating on the polymer layer.

17. Sensor and guide wire assembly according to claim 15, **characterized in that** the hydrophilic agent or material is incorporated in the polymer layer.

18. Sensor and guide wire assembly according to claim 15, **characterized in that** the polymer layer consists of a hydrophilic material.

19. Sensor and guide wire assembly according to claim 1, **characterized in that** the polymer layer is reinforced with another material.

20. Sensor and guide wire assembly according to claim 19, **characterized in that** the polymer layer is braided with metal threads.

21. Sensor and guide wire assembly according to claim 1, **characterized in that** the polymer layer, the core wire, and said at least one signal transmitting cable are co-extruded.

## Patentansprüche

1. Sensor- und Führungsdrahtanordnung für intravaskuläre Messungen einer physiologischen Variablen in einem lebenden Körper, die ein Sensorelement (22, 42) und einen Sensorführungsdraht mit einem Kerndraht (28, 48) und zumindest einem Signalübertragungskabel (31, 51), das mit dem Sensorelement verbunden ist, umfasst, wobei das zumindest eine Signalübertragungskabel (11, 21, 31) entlang des Kerndrahts bis zum proximalen Endabschnitt verläuft, wobei der Sensorführungsdraht ferner eine proximale Röhre (29, 49), eine distale Spitze (24, 44) und eine Polymerschicht (27, 47), die einen Teil des Kerndrahts und das zumindest eine Signalübertragungskabel umschließt, umfasst, wobei sich die Polymerschicht zwischen der distalen Spitze und der proximalen Röhre erstreckt und **dadurch gekennzeichnet, dass** das Signalübertragungskabel elektrisch mit einem leitenden Element (32, 52) verbunden ist, um eine proximale Steckerverbindung zu bilden.

2. Sensor- und Führungsdrahtanordnung nach Anspruch 1, **dadurch gekennzeichnet, dass** die Polymerschicht in Form einer Röhre oder Hülse vorgesehen ist.

3. Sensor- und Führungsdrahtanordnung nach Anspruch 1, **dadurch gekennzeichnet, dass** die Polymerschicht in Form eines Überzugs vorgesehen ist.

4. Sensor- und Führungsdrahtanordnung nach Anspruch 1, **dadurch gekennzeichnet, dass** der Sensorführungsdraht ferner ein Sensorgehäuse (26) umfasst und die Polymerschicht zwischen dem Sensorgehäuse und der proximalen Röhre verläuft.

5. Sensor- und Führungsdrahtanordnung nach Anspruch 4, **dadurch gekennzeichnet, dass** das Sensorgehäuse in Form eines Mantels oder einer Hülse vorgesehen ist.

6. Sensor- und Führungsdrahtanordnung nach Anspruch 4, **dadurch gekennzeichnet, dass** die Polymerschicht das Sensorgehäuse bildet.

7. Sensor- und Führungsdrahtanordnung nach Anspruch 1, **dadurch gekennzeichnet, dass** die Polymerschicht proximal zum Sensorelement stattdessen eine Metallröhre ist.

8. Sensor- und Führungsdrahtanordnung nach Anspruch 7, **dadurch gekennzeichnet, dass** die Metallröhre aus Edelstahl besteht.

9. Sensor- und Führungsdrahtanordnung nach Anspruch 1, **dadurch gekennzeichnet, dass** der Sensorführungsdraht ferner eine distale Spiralfeder (25, 45) umfasst und die Polymerschicht zwischen der distalen Spiralfeder und der proximalen Röhre verläuft.

10. Sensor- und Führungsdrahtanordnung nach Anspruch 1, **dadurch gekennzeichnet, dass** das zumindest eine Signalübertragungskabel (71) in der Polymerschicht eingebettet ist.

11. Sensor- und Führungsdrahtanordnung nach Anspruch 1, **dadurch gekennzeichnet, dass** die Polymerschicht ein reibungsarmes Material umfasst.

12. Sensor- und Führungsdrahtanordnung nach Anspruch 11, **dadurch gekennzeichnet, dass** das reibungsarme Material als Überzug auf der Polymerschicht aufgebracht wird.

13. Sensor- und Führungsdrahtanordnung nach Anspruch 11, **dadurch gekennzeichnet, dass** das reibungsarme Material in die Polymerschicht eingearbeitet ist.

14. Sensor- und Führungsdrahtanordnung nach Anspruch 11, **dadurch gekennzeichnet, dass** die Polymerschicht aus einem reibungsarmen Material besteht.

15. Sensor- und Führungsdrahtanordnung nach Anspruch 1, **dadurch gekennzeichnet, dass** die Polymerschicht ein hydrophiles Mittel oder Material umfasst.

16. Sensor- und Führungsdrahtanordnung nach Anspruch 15, **dadurch gekennzeichnet, dass** das hydrophile Mittel oder Material als Überzug auf der Polymerschicht aufgebracht wird.

17. Sensor- und Führungsdrahtanordnung nach Anspruch 15, **dadurch gekennzeichnet, dass** das hydrophile Mittel oder Material in die Polymerschicht eingearbeitet ist.

18. Sensor- und Führungsdrahtanordnung nach Anspruch 15, **dadurch gekennzeichnet, dass** die Polymerschicht aus einem hydrophilen Material besteht.

19. Sensor- und Führungsdrahtanordnung nach Anspruch 1, **dadurch gekennzeichnet, dass** die Polymerschicht mit einem anderen Material verstärkt ist.

20. Sensor- und Führungsdrahtanordnung nach Anspruch 19, **dadurch gekennzeichnet, dass** die Polymerschicht mit Metallfäden geflochten ist.

21. Sensor- und Führungsdrahtanordnung nach Anspruch 1, **dadurch gekennzeichnet, dass** die Polymerschicht, der Kerndraht und das zumindest eine Signalübertragungskabel coextrudiert sind.

## Revendications

1. Ensemble de détecteur et de fil de guidage destiné à la mesure intravasculaire d'une variable physiologique d'un corps vivant, comprenant
un élément de détection (22, 42) et un fil de guidage de détecteur qui comprend un fil d'âme (28, 48) et au moins un câble (31, 51) de transmission de signaux relié à l'élément de détection,
ledit ou lesdits câbles (11, 21, 31) de transmission de signaux s'étendant le long du fil d'âme jusqu'à la partie d'extrémité proximale,
le fil de guidage de détecteur comprenant en outre un tube proximal (29, 49), une pointe distale (24, 44) et une couche de polymère (27, 47) qui englobe une partie du fil de guidage et dudit ou desdits câbles de transmission de signaux,
la couche de polymère s'étendant entre la pointe distale et le tube proximal,
**caractérisé en ce que**
le câble de transmission de signaux est relié électriquement à un élément conducteur (32, 52) de manière à former un connecteur mâle proximal.

2. Ensemble de détecteur et de fil de guidage selon la revendication 1, **caractérisé en ce que** la couche de polymère est prévue sous la forme d'un tube ou d'un manchon.

3. Ensemble de détecteur et de fil de guidage selon la revendication 1, **caractérisé en ce que** la couche de polymère est prévue sous la forme d'un revêtement.

4. Ensemble de détecteur et de fil de guidage selon la revendication 1, **caractérisé en ce que** le fil de guidage de détecteur comprend en outre un logement de détecteur (26) et **en ce que** la couche polymère s'étend entre le logement de détecteur et le tube proximal.

5. Ensemble de détecteur et de fil de guidage selon la revendication 4, **caractérisé en ce que** le logement de détecteur est prévu sous la forme d'une jaquette ou d'un fourreau.

6. Ensemble de détecteur et de fil de guidage selon la revendication 4, **caractérisé en ce que** la couche de polymère forme le logement du détecteur.

7. Ensemble de détecteur et de fil de guidage selon la revendication 1, **caractérisé en ce qu'**un tube métallique remplace la couche de polymère à proximité de l'élément de détecteur.

8. Ensemble de détecteur et de fil de guidage selon la revendication 7, **caractérisé en ce que** le tube métallique est réalisé en acier inoxydable.

9. Ensemble de détecteur et de fil de guidage selon la revendication 1, **caractérisé en ce que** le fil de guidage du détecteur comprend en outre un ressort hélicoïdal distal (25, 45) et **en ce que** la couche de polymère s'étend entre le ressort hélicoïdal distal et le tube proximal.

10. Ensemble de détecteur et de fil de guidage selon la revendication 1, **caractérisé en ce que** ledit ou lesdits câbles (71) de transmission de signaux sont incorporés dans la couche de polymère.

11. Ensemble de détecteur et de fil de guidage selon la revendication 1, **caractérisé en ce que** la couche de polymère contient un matériau à bas frottement.

12. Ensemble de détecteur et de fil de guidage selon la revendication 11, **caractérisé en ce que** le matériau à bas frottement est appliqué sous la forme d'un revêtement sur la couche de polymère.

13. Ensemble de détecteur et de fil de guidage selon la revendication 11, **caractérisé en ce que** le matériau à bas frottement est incorporé dans la couche de polymère.

14. Ensemble de détecteur et de fil de guidage selon la revendication 11, **caractérisé en ce que** la couche de polymère est constituée d'un matériau à bas frottement.

15. Ensemble de détecteur et de fil de guidage selon la revendication 1, **caractérisé en ce que** la couche de polymère contient un agent ou matériau hydrophile.

16. Ensemble de détecteur et de fil de guidage selon la revendication 15, **caractérisé en ce que** l'agent ou matériau hydrophile est appliqué sous la forme d'un revêtement sur la couche de polymère.

17. Ensemble de détecteur et de fil de guidage selon la revendication 15, **caractérisé en ce que** l'agent ou matériau hydrophile est incorporé dans la couche de polymère.

18. Ensemble de détecteur et de fil de guidage selon la revendication 15, **caractérisé en ce que** la couche de polymère est constituée d'un matériau hydrophile.

19. Ensemble de détecteur et de fil de guidage selon la revendication 1, **caractérisé en ce que** la couche de polymère est renforcée par un autre matériau.

20. Ensemble de détecteur et de fil de guidage selon la revendication 19, **caractérisé en ce que** des fils métalliques sont tressés dans la couche de polymère.

21. Ensemble de détecteur et de fil de guidage selon la revendication 1, **caractérisé en ce que** la couche de polymère, le fil d'âme et ledit ou lesdits câbles de transmission de signaux sont co-extrudés.
